# EUROPÄISCHE PATENTSCHRIFT

(11) **EP 0 719 535 B1**
(45) Veröffentlichungstag und Bekanntmachung des Hinweises auf die Patenterteilung: **02.08.2000**
(21) Anmeldenummer: 95119177.4
(22) Anmeldetag: 06.12.1995
(51) Int. Cl.: A61F 15/00

(54) **Austragvorrichtung für Watterollen oder ähnliches**
Dispenser for cotton-wool rolls or similar
Distributeur pour rouleaux d'ouate

(30) Priorität: 30.12.1994 DE 4447188
(43) Veröffentlichungstag der Anmeldung: 03.07.1996
(73) Patentinhaber: ROEKO GmbH + Co. Dentalerzeugnisse, 89122 Langenau (DE)
(72) Erfinder: Loos, Ralf, D-81447 München (DE)
(74) Vertreter: Engelhardt, Guido, Dipl.-Ing.

(56) Entgegenhaltungen:
- DE-U- 9 319 912
- FR-A- 2 251 197
- FR-E- 46 483
- US-A- 2 342 747

## Beschreibung

Die Erfindung bezieht sich auf eine Vorrichtung zur Ausbringung von in einem quaderförmigen Gehäuse eingelagerten Watterollen oder ähnlichen stangenartigen Werkstücken nach dem Oberbegriff der Patentansprüche 1 und 2.

Aus der FR 46.483 ist ein solcher Wattenrollenspender bekannt. Die verstellbare Bodenplatte ist hierbei an einem Bolzen an einer der Auslaßöffnung gegenüberliegenden Gehäusewand des Watterollenspenders angelenkt und ragt teilweise aus der Auslaßöffnung des Gehäuses heraus. Die Watterollen liegen frei beweglich auf der Bodenplatte auf. Unterhalb der Bodenplatte ist eine drehbewegliche Wippe angeordnet, die einen an der Bodenplatte anliegenden Buckel aufweist. Auf der der Auslaßöffnung gegenüberliegenden Wand des Gehäuses ragt die Wippe zu Betätigungszwecken heraus.

Drückt ein Benutzer die Wippe zur Ausbringung einer Watterollen hinunter, so verändert sich der Neigungswinkel der Bodenplatte derart, daß zum einen die Watterollen in Richtung der Auslaßöffnung rollen und zum anderen eine Durchlaßöffnung zwischen einer oberhalb der Auslaßöffnung vorgesehenen Wand des Gehäuses und der Bodenplatte entsteht, durch die die am nächsten zur Auslaßöffnung liegende Watterolle durch paßt.

Nach dem Loslassen der Wippe wird die Bodenplatte mittels einer Druckfeder in die Ausgangslage zurück überführt, so daß die Auslaßöffnung wieder verschlossen ist und die Watterollen an der Wand des Gehäuses anliegen.

Aus der US 2,342,747 ist ein Behälter zur Aufbewahrung von Zigarretten bekannt geworden, der eine mit einem Fach für eine Zigarrette ausgestattete Schublade aufweist, mittels der die Zigaretten aus dem Behälter einzeln ausbringbar sind.

Als nachteilig bei dem Stand der Technik gemäß der FR 46.483 hat es sich gezeigt, daß in diese Spende-Vorrichtung nur Watterollen gelagert und heraus gelassen werden können, deren Durchmesser in etwa so groß bemessen ist, daß nur die Watterolle durch die Durchlaßöffnung passen, die zwischen der neben der Auslaßöffnung angeordneten Wand des Gehäuses und der Bodenplatte durch Hinunterdrücken der Wippe entsteht.
Falls die Watterollen in ihrem Durchmesser kleiner oder größer bemessen sind, rollen beim Herunterdrücken der Wippe entweder zuviele Watterollen durch die Durchlaßöffnung oder die zu große Watterolle paßt nicht durch die Durchlaßöffnung, so daß demnach die Spende-Vorrichtung nicht funktioniert.

Des weiteren ist die Betätigung dieser Spende-Vorrichtung umständlich und daher zeitaufwendig, da die Wippe auf der Rückseite des Gehäuses angeordnet ist, so daß der Benutzer um das Gehäuse fassen muß, um die Wippe zu betätigen. Anschließend gelangt die einzelne Watterolle auf der der Wippe gegenüberliegenden Seite aus dem Gehäuse, so daß der Benutzer wiederum um das Gehäuse fassen muß, um die Watterolle aufnehmen zu können.

Aufgabe der Erfindung ist es daher, eine Vorrichtung der eingangs genannten Gattung zu schaffen, die nicht nur äußerst einfach in ihrer Handhabung ist, sondern bei der vor allem sichergestellt ist, daß verschieden groß bemessene Watterollen leicht dem Gehäuse entnommen werden können, aber dennoch vor einer evtl. Verunreinigung zuverlässig geschützt sind. Der dazu erforderliche Bauaufwand soll gering gehalten werden, auch soll eine vielseitige Verwendbarkeit bei leichter Bedienbarkeit gegeben sein.

Gemäß der Erfindung werden diese Aufgaben durch die Merkmale im kennzeichnenden Teil der Patentansprüche 1 und/ oder 2 gelöst.

Der Boden kann mit einem Ende an dem Gehäuse angelenkt oder höhenverstellbar in dem Gehäuse geführt sein, wobei es zweckmäßig ist, den Boden in Form eines den Aufnahmeraum des Gehäuses begrenzenden Winkelstückes auszubilden und mit dem der Auslaßöffnung gegenüberliegenden Schenkel im oberen Randbereich des Gehäuses an diesem, z. B. mittels eines Gelenkbolzens, verschwenkbar zu befestigen.

Es ist auch möglich, das Verstellglied durch einen an dem Boden, vorzugsweise im Bereich des diesen tragenden Gelenkbolzens angebrachten, aus dem Gehäuse ragenden Griffstückes, zu bilden.

Angebracht ist es ferner, den Boden auf der die Auslaßöffnung durchgreifenden Seite mit einer vor dem Gehäuse befindlichen angeformten Aufnahmewanne für eine Watterolle oder das Gehäuse mit einer vor der Auslaßöffnung angeordneten ortsfesten Aufnahmewanne zu versehen.

Des weiteren ist es vorteilhaft, wenn der Boden im Bereich vor der Auslaßöffnung des Gehäuses mit einer vorzugsweise konvex gekrümmt ausgebildeten in den Aufnahmeraum ragenden Ausbiegung versehen ist, die in einem Abstand zur Auslaßöffnung angeordnet sein sollte, der etwa dem Durchmesser der in dem Gehäuse eingelagerten Watterollen entspricht, und wenn auf einer oder beiden Innenwänden des Gehäuses ein oder mehrere, sich vorzugsweise über den Verstellbereich des Bodens erstreckende und keilförmig ausgebildete Vorsprünge vorgesehen sind.

Die dem Anschlag zugekehrte mit dem Boden zusammenwirkende, die Auslaßöffnung begrenzende Wand des Gehäuses sollte als nach außen geneigte Schrägfläche ausgebildet sein, auch kann an dem Anschlag auf der dem Gehäuse abgekehrten Seite eine Auffangwanne angebracht sein.

Die auf den Boden des Gehäuses einwirkenden Rückstellfedern sollten bei einem als Winkelstück ausgebildeten Boden zwischen dessen angelenktem Schenkel und dem Gehäuse eingesetzt werden, wobei die Rückstellfedern auch durch an dem Gehäuse angespritzte Blattfedern gebildet sein können.

Wird eine Vorrichtung zur Ausbringung von eingelagerten Watterollen gemäß der Erfindung ausgebildet, so ist sichergestellt, daß die in dem Gehäuse bevorrateten Watterollen nicht nur ohne Schwierigkeiten dem Gehäuse zu entnehmen sind , sondern auch, daß die in diesem befindlichen Watterollen vor Verunreinigungen geschützt sind. Zur Entnahme von Watterollen ist nämlich lediglich das Verstellglied, beispielsweise der Deckel des Gehäuses, zu betätigen, um durch den mit diesem trieblich verbundenen Boden eine Watterolle auszustoßen. Und da der Boden die Auslaßöffnung durchgreift und somit an einer Wand des Gehäuses anliegt, wird diese nur für eine kurze Zeitspanne während der Verstellung des Bodens geöffnet. Auch kann die aus dem Gehäuse ausgestoßene und in eine Auffangwanne eingebrachte Watterolle nicht in das Gehäuse zurückgeführt werden, eine Verschmutzung der eingelagerten Watterollen ist demnach weitgehend ausgeschlossen.

Darüber hinaus kann die Durchlaßöffnung zur Ausbringung der eingelagerten Watterollen manuell eingestellt und somit exakt an den Durchmesser der eingelagerten Watterollen angepaßt werden, so daß ein Verstopfen des Durchlaßes oder das gleichzeitige Ausbringen von mehreren Watterollen vermieden wird.
Außer der einfachen Handhabung des vorschlagsgemäß ausgebildeten Spenders ist demnach von Vorteil, daß dieser in sehr vielseitiger Weise auch für im Durchmesser unterschiedlich bemessene Watterollen verwendbar und für dessen Herstellung nur ein geringer Bau- und Fertigungsaufwand erforderlich ist. Das Gehäuse und die weiteren Bauteile können nämlich in wirtschaftlicher Weise aus Kunststoff gefertigt werden. Die Entnahme jeweils nur einer in einem Gehäuse eingelagerten Watterollen oder ähnlichen Werkstücken wird somit aufgrund der erfindungsgemäßen Ausgestaltung wesentlich erleichtert.

Die einfache und schnelle Bedienbarkeit der Austragvorrichtung ist dadurch gewährleistet, daß die Bodenplatte unmittelbar mittels der Deckelplatte abgesenkt werden kann, so daß der Benutzer lediglich eine Bewegung, nämlich von oben nach unten, vollziehen muß, um eine Watterrolle aus dem Gehäuse heraus zu lassen und diese anschließend von der Auslaßöffnung aufnehmen zu können.

In der Zeichnung ist ein Ausführungsbeispiel der gemäß der Erfindung ausgebildeten Vorrichtung zur Ausbringung von Watterollen dargestellt, das nachfolgend im einzelnen erläutert ist. Hierbei zeigt:
- Figur 1: die Vorrichtung in einem achssenkrechten Schnitt, in Ruhestellung,
- Figur 2: die Vorrichtung nach Figur 1, in Draufsicht,
- Figuren 3 und 4: die Vorrichtung nach Figur 1, in anderen Betriebsstellungen, und
- Figur 5: die Vorrichtung nach Figur 1, in einer anderen Ausführungsvariante.

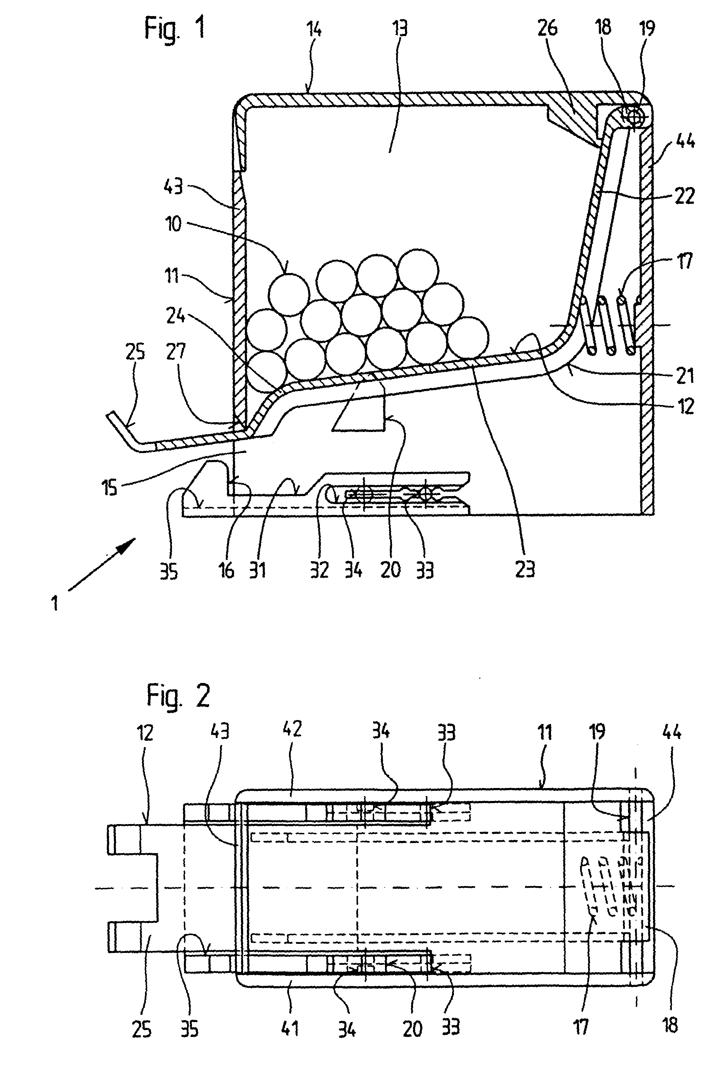

Die in den Figuren 1 bis 5 dargestellte und mit 1 bzw. 1' bezeichnete Vorrichtung dient zur Ausbringung von in einem quaderförmig ausgebildeten Gehäuse 11 in einem Aufnahmeraum 13 eingelagerten Watterollen 10, die einer in einer Seitenwand 43 des Gehäuses 11 vorgesehenen Auslaßöffnung 15 entnehmbar sind. Dazu dient ein höhenverstellbar in dem Gehäuse 11 angeordneter Boden 12, der den Aufnahmeraum 13 begrenzt und bei der Ausgestaltung nach den Figuren 1 bis 4 mit Hilfe eines auf dem Gehäuse 11 aufgesetzten Deckels 14 betätigbar ist.

Der Boden 12 ist bei dem gezeigten Ausführungsbeispiel als Winkelstück 21 ausgebildet, dessen vertikal gerichteter Schenkel 22 mittels eines in an dem Gehäuse 11 angebrachter Laschen 18 abgestützten Gelenkbolzens 19, auf dem auch der Deckel 14 hochklappbar gelagert ist, an einer Wand 44 des Gehäuses 11 im oberen Bereich verschwenkbar gehalten ist. Des weiteren ist zwischen dem Schenkel 22 und der Wand 44 des Gehäuses 11 eine Rückstellfeder 17 eingesetzt, so daß der etwa horizontal verlaufende Schenkel 23 des Winkelstückes 21, der die in der gegenüberliegenden Wand 43 des Gehäuses 11 eingearbeitete Auslaßöffnung 15 durchgreift, ständig an der Wand 43 anliegt. Ferner ist der Schenkel 23 mit einer konvex gekrümmt ausgebildeten Ausbiegung 24 versehen, die mit einem derartigen Abstand zur Auslaßöffnung 15 angeordnet ist, daß jeweils nur eine der eingelagerten Watterollen 10 ausbringbar ist.

Der Auslaßöffnung 15 ist ferner ein Anschlag 16 in Form eines Höckers zugeordnet, der bei der Ausgestaltung nach den Figuren 1 bis 5 an einem verstellbaren Schieber 31 angeformt ist. Um dies bewerkstelligen zu können, sind an den Seitenwänden 41 und 42 des Gehäuses 11 jeweils zwei Bolzen 33 und 34 nach innen abstehend angeformt und der Schieber 31 ist mit einer langlochartigen, in Achsrichtung geschlitzten und abgesetzten Ausnehmung 32 versehen. Der Schieber 31 kann somit in unterschiedlichen Stellungen, die im Durchmesser unterschiedlich bemessenen Watterollen zuzuordnen sind, arretiert werden, außerdem wird durch die Bolzen 34 verhindert, daß der Schieber 31 versehentlich in das Gehäuse 11 eingeschoben wird.

An dem Schenkel 23 des Winkelstückes 21 und somit an dem Boden 11 ist ferner eine Auffangwanne 25 angformt, und der Anschlag 16 weist eine entsprechende Freisparung 35 auf, so daß die Aufnahmewanne 25 durch den Anschlag 16 abgesenkt werden kann. Und um zu verhindern, daß sich Watterollen 10 in dem Aufnahmeraum 13 querlegen oder Brücken bilden, sind an den Seitenwänden 41 und 42 nach innen abstehende keilförmig ausgebildete Vorsprünge 20 angeformt, die sich über den Verstellbereich des verschwenkbar gelagerten Bodens 12 erstrecken.

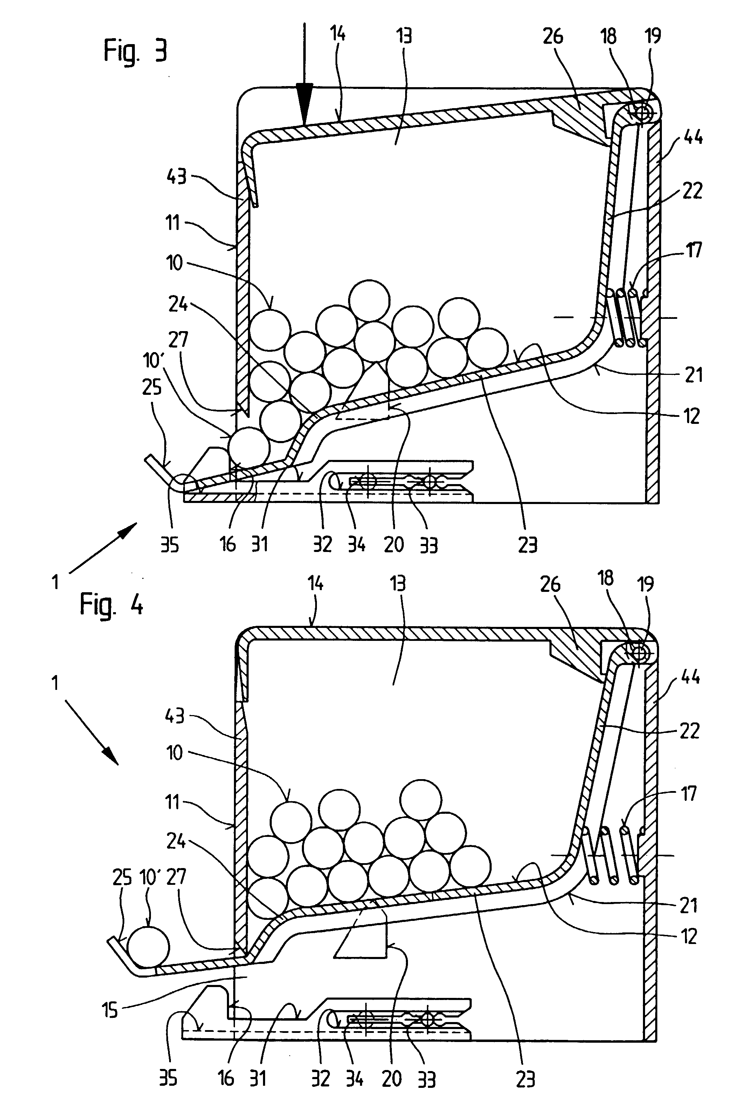

Wird der Deckel 14 des Gehäuses 11, wie dies in Figur 3 dargestellt ist, von Handniedergedrückt, so wird der Boden 12, da auf der Innenseite des Deckels 14 eine auf den Boden 12 einwirkende Rippe 26 angebracht und der Deckel 14 somit trieblich mit dem Boden 12 verbunden ist, entgegen der Kraft der Rückstellfeder 17 um den Gelenkbolzen 19 geschwenkt und ebenfalls nach unten verstellt. Dadurch kann eine der eingelegten Watterollen 10' sich an dem Anschlag 16 anlegen. Wird auf den Deckel 14 keine Kraft mehr ausgeübt, werden durch die Kraft der Rückstellfeder 17 der Boden 12 sowie der Deckel 14 gemäß Figur 4 in die Ausgangslage selbsttätig zurückgeführt. Die an dem Anschlag 16 anliegende Watterolle 10' rollt dabei in die an dem Schenkel 23 vorgesehene Aufnahmewanne 25 und kann somit leicht der Vorrichtung 1 entnommen werden. Durch die Ausbildung der dem Anschlag 16 zugekehrten Fläche der Auslaßöffnung 15 als Schrägfläche 27 wird die Ausbringung der Watterolle 10' begünstigt.

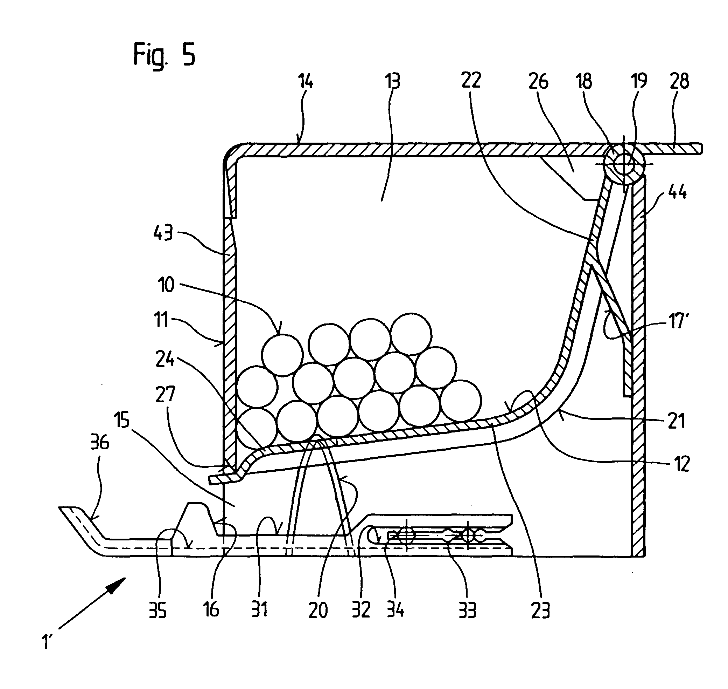

Bei der Ausführungsvariante der Vorrichtung 1' nach Figur 5 ist als auf den Boden 12 einwirkendes Verstellglied ein Griffstück 28 vorgesehen, das unmittelbar an dem Boden 12 im Bereich des Gelenkbolzens 19 angebracht ist und aus dem Gehäuse 11 herausragt. Die auf den Boden 12 einwirkende Rückstellfeder 17' ist bei dieser Ausgestaltung als an dem Schenkel 22 des Bodens 12 angespritzte Blattfeder ausgebildet. Außerdem ist der Schenkel 23 des Winkelstückes 21 verkürzt ausgebildet und an dem Schieber 31 ist eine ortsfeste Aufnahmewanne 36 angeformt, die aus der Vorrichtung 1' einzeln zu entnehmenden Watterollen 10 werden bei der Rückführung des Bodens 12 somit durch diesen über den Anschlag 16 gehoben und rollen in die Aufnahmewanne 36.

## Patentansprüche

1. Vorrichtung (1) zur Ausbringung von in einem quaderförmigen Gehäuse (11) eingelagerten Watterollen (10) oder ähnlichen stangenartigen Werkstücken, wobei das Gehäuse (11) auf einer der Schmalseiten (43) eine parallel zu den eingelagerten Watterollen (10) angeordnete Auslaßöffnung (15) aufweist und der die Watterollen (10) tragende Boden (12) des Gehäuses (11) entgegen der Kraft einer oder mehrerer Rückstellfedern (17, 17') höhenverstellbar in dem Gehäuse (11) angeordnet ist und mit einer Schmalseite die Auslaßöffnung (15) ganz oder teilweise durchgreift,
**dadurch gekennzeichnet,**
daß die Auslaßöffnung (15) des Gehäuses (11) mit einem vor dieser auf der Unterseite angeordneten vorzugsweise als Höcker ausgebildeten Anschlag (16) versehen ist, daß der Anschlag (16) in Längsrichtung des Gehäuses (11) verstellbar mit diesem verbunden ist und daß der Anschlag (16) an einem Schieber (31) angeformt ist, der eine langlochartige Ausnehmung (32) aufweist und mittels an dem Gehäuse (11) angeformter in die Ausnehmung (32) eingreifender Rastnocken (33, 34) in unterschiedlichen Abständen zu der Auslaßöffnung (15) arretierbar ist.

2. Vorrichtung (1) zur Ausbringung von in einem quaderförmigen Gehäuse (11) eingelagerten Watterollen (10) oder ähnlichen stangenartigen Werkstücken, wobei das Gehäuse (11) auf einer der Schmalseiten (43) eine parallel zu den eingelagerten Watterollen (10) angeordnete Auslaßöffnung (15) aufweist und der die Watterollen (10) tragende Boden (12) des Gehäuses (11) entgegen der Kraft einer oder mehrerer Rückstellfedern (17, 17') höhenverstellbar in dem Gehäuse (11) angeordnet ist und mit einer Schmalseite die Auslaßöffnung (15) ganz oder teilweise durchgreift, und auch nach Anspruch 1,
**dadurch gekennzeichnet,**
daß zur Höhenverstellung des Bodens (12) als Verstellglied der Deckel (14) des Gehäuses (11) vorgesehen ist, der unmittelbar oder über Zwischenglieder (26) auf diesen einwirkt oder an diesem angebracht ist.

3. Vorrichtung nach Anspruch 1 oder 2,
**dadurch gekennzeichnet,**
daß der Boden (12) mit einem Ende an dem Gehäuse (11) angelenkt oder höhenverstellbar in dem Gehäuse (11) geführt ist.

4. Vorrichtung nach einem oder mehreren der vorgenannten Ansprüche,
**dadurch gekennzeichnet,**
daß der Boden (12) in Form eines den Aufnahmeraum (13) des Gehäuses (11) begrenzenden Winkelstückes (21) ausgebildet ist und mit dem der Auslaßöffnung (15) gegenüberliegenden Schenkel (22) im oberen Randbereich des Gehäuses (11) an diesem mittels eines Gelenkbolzens (19) verschwenkbar angelenkt ist.

5. Vorrichtung nach Anspruch 2,
**dadurch gekennzeichnet,**
daß als Verstellglied ein auf dem Gehäuse (11) aufgesetzter Deckel (14) vorgesehen ist, der zusammen mit dem Boden (12) an dem Gehäuse (11) angelenkt ist und mittels eines angeformten Vorsprunges (26) oder dgl. als Zwischenglied mit dem Boden (12) zusammenwirkt.

6. Vorrichtung nach Anspruch 5,
**dadurch gekennzeichnet,**
daß das Verstellglied durch ein an dem Boden (12) vorzugsweise im Bereich des diesen tragenden Gelenkbolzens (19) angebrachten aus dem Gehäuse (11) ragenden Griffstück (28) gebildet ist.

7. Vorrichtung nach einem oder mehreren der Ansprüche 1 bis 6,
**dadurch gekennzeichnet,**
daß der Boden (12) auf der die Auslaßöffnung (15) durchgreifenden Seite mit einer angeformten vor dem Gehäuse (11) befindliche Aufnahmewanne (25) für eine Watterolle (10') versehen ist.

8. Vorrichtung nach einem oder mehreren der Ansprüche 1 bis 6,
**dadurch gekennzeichnet,**
daß das Gehäuse (11) mit einer vor der Auslaßöffnung (15) angeordneten ortsfesten Aufnahmewanne (34) versehen ist.

9. Vorrichtung nach einem oder mehreren der Ansprüche 1 bis 8,
**dadurch gekennzeichnet,**
daß der Boden (12) im Bereich vor der Auslaßöffnung (15) des Gehäuses (11) mit einer vorzugsweise konvex gekrümmt ausgebildeten in den Aufnahmeraum (13) ragenden Ausbiegung (24) versehen ist.

10. Vorrichtung nach Anspruch 9,
**dadurch gekennzeichnet,**
daß die in dem Boden (12) vorgesehene Ausbiegung (24) mit einem Abstand zur Auslaßöffnung (15) angeordnet ist, der etwa dem Durchmesser der in dem Gehäuse (11) eingelagerten Watterollen (10) entspricht.

11. Vorrichtung nach einem oder mehreren der Ansprüche 1 bis 10,
**dadurch gekennzeichnet,**
daß auf einer oder beiden Seitenwänden (41, 42) des Gehäuses (11) auf den Innenseiten ein oder mehrere, sich vorzugsweise über den Verstellbereich des Bodens (12) erstreckende und keilförmig ausgebildete Vorsprünge (20) vorgesehen sind.

12. Vorrichtung nach Anspruch 1,
**dadurch gekennzeichnet,**
daß der Anschlag (16) mit einer die an dem Boden (12) vorgesehene Aufnahmewanne (25) aufnehmenden Freisparung (36) versehen ist.

13. Vorrichtung nach Anspruch 1,
**dadurch gekennzeichnet,**
daß die dem Anschlag (16) zugekehrte mit dem Boden (12) zusammenwirkende die Auslaßöffnung (15) begrenzende Wand des Gehäuses (11) als nach außen geneigte Schrägfläche (27) ausgebildet ist.

14. Vorrichtung nach Anspruch 1, 12 oder 13,
**dadurch gekennzeichnet,**
daß an dem Anschlag (16) auf der dem Gehäuse (11) abgekehrten Seite eine Auffangwanne (36) angebracht ist.

15. Vorrichtung nach einem oder mehreren der Ansprüche 1 bis 14,
**dadurch gekennzeichnet,**
daß die auf den Boden (12) des Gehäuses (11) einwirkenden Rückstellfedern (17) bei einem als Winkelstück (21) ausgebildeten Boden (12) zwischen dessen angelenktem Schenkel (22) und dem Gehäuse (11) eingesetzt sind.

16. Vorrichtung nach einem oder mehreren der Ansprüche 1 bis 15,
**dadurch gekennzeichnet,**
daß die auf den Boden (12) einwirkende Rückstellfeder (17') als an diesem angespritzte Blattfeder ausgebildet ist.

## Claims

1. A device (1) for dispensing cotton wool rolls (10) or similar roll-shaped workpieces stored in a rectangular box-shaped housing (11), in which one of the narrow sides (43) of the housing (11) has an outlet opening (15) arranged in parallel to the cotton wool rolls (10) and the base (12) of the housing (11) which carries the cotton wool rolls (10) is held in the housing (11) at a movable height against the force of one or more return springs (17, 17') and one narrow side of the base (12) projects wholly or in part through the outlet opening (15),
**characterised in that**,
the outlet opening (15) of the housing (11) is equipped with a stop (16) configured, in a preferred embodiment, as a step located in front of and on the underside of the outlet opening (15), that the stop (16) is connected to the housing (11) and can be moved in the lengthways direction and that the stop (16) is formed on a slide (31) having a slot-like recess (32) and can be locked at different distances from the outlet opening (15) by means of detent cams (33, 34) formed onto the housing (11) and engaging in the recess (32).

2. A device (1) for dispensing cotton wool rolls (10) or similar roll-shaped workpieces stored in a rectangular box-shaped housing (11), in which one of the narrow sides (43) of the housing (11) has an outlet opening (15) arranged in parallel to the cotton wool rolls (10) and the base (12) of the housing (11) which carries the cotton wool rolls (10) is held in the housing (11) at a movable height against the force of one or more return springs (17, 17') and one narrow side of the base (12) projects wholly or in part through the outlet opening (15), and also in accordance with Claim 1,
**characterised in that**,
the cover (14) of the housing (11) is provided as the actuator for moving the height of the base (12), with the cover (14) acting on the base (12) either directly or via an intermediary element (26) or being attached to the base (12).

3. The device in accordance with Claim 1 or 2,
**characterised in that**,
one end of the base (12) is attached to the housing (11) or is guided in the housing (11) with a movable height.

4. The device in accordance with one or more of the aforementioned Claims,
**characterised in that**,
the base (12) is shaped like an elbow (21) which delimits the storage area (13) of the housing (11) and the leg (22) of the base (12) opposite to the outlet opening (15) is attached to the housing (11) in the upper edge zone of the housing (11) in a pivoting arrangement by means of an articulated pin (19).

5. The device in accordance with Claim 2,
**characterised in that**,
an actuator is provided in the form of a cover (14) placed on the housing (11), with this cover (14) being attached to the housing (11) together with the base (12) and acting in conjunction with the base (12) by means of a projection (26) or the like which is formed onto the cover (14) and which functions as an intermediary element.

6. The device in accordance with Claim 5,
**characterised in that**,
the actuator is formed by a handle (28) projecting from the housing (11), with the actuator being attached to the base (12) in, in a preferred embodiment, the area of the articulated pin (19) which carries the base (12).

7. The device in accordance with one or more of Claims 1 to 6,
**characterised in that**,
the side of the base (12) which protrudes through the outlet opening (15) is provided with a collecting tray (25) for a cotton wool roll (10'), the collecting tray (25) being formed onto the base (12) in front of the housing (11).

8. The device in accordance with one or more of Claims 1 to 6,
**characterised in that**,
the housing (11) is provided with a fixed collecting tray (34) located in front of the outlet opening (15).

9. The device in accordance with one or more of Claims 1 to 8,
**characterised in that**,
the area of the base (12) in front of the outlet opening (15) of the housing (11) is provided with a bend (24) projecting into the storage area (13), with the bend (24) having, in a preferred embodiment, a convex curvature.

10. The device in accordance with Claim 9,
**characterised in that**,
the bend (24) provided in the base (12) is arranged at a distance from the outlet opening, with this distance approximately corresponding to the diameter of the cotton wool rolls (10) stored in the housing (11).

11. The device in accordance with one or more of Claims 1 to 10,
**characterised in that**,
one or more projections (20), in a preferred embodiment extending across the movement range of the base (12) and having a wedge shape, are provided on the insides of one or both of the side walls (41, 42) of the housing (11).

12. The device in accordance with Claim 1,
**characterised in that**,
the stop (16) is provided with a recess (36) which accommodates the collecting tray (25) provided on the base (12).

13. The device in accordance with Claim 1,
**characterised in that**,
the wall of the housing (11) which faces the stop (16), acts in conjunction with the base (12) and delimits the outlet opening (15) is formed as an inclined surface (27) slanting outwards.

14. The device in accordance with Claim 1, 12 or 13,
**characterised in that**,
a collecting tray (36) is attached to the side of the stop (16) facing away from the housing (11).

15. The device in accordance with one or more of Claims 1 to 14,
**characterised in that**,
the return springs (17) acting on the base (12) of the housing (11) are inserted between the angled leg (22) of the base (12) and the housing (11) when the base (12) is shaped like an elbow (21).

16. The device in accordance with one or more of Claims 1 to 15,
**characterised in that**,
the return spring (17') acting on the base (12) is formed as a leaf spring moulded onto the base (12).

## Revendications

1. Dispositif (1) de distribution de rouleaux de ouate (10) ou de pièces similaires en barre, logés dans un boîtier cube (11), le boîtier (11) comportant sur un des côtés étroits (43) une ouverture de distribution (15) disposée parallèlement aux rouleaux de ouate enfermés (10), et le fond (12) du boîtier (11) portant les rouleaux de ouate (10) étant réglable en hauteur dans le boîtier (11), ceci contre la force d'un ou de plusieurs ressorts de rappel (17, 17'), et dont un côté étroit passe partiellement ou entièrement par l'ouverture de distribution (15),
caractérisé en ce que
l'ouverture de distribution (15) du boîtier (11) est munie d'une butée (16) disposée en bas devant celle-ci et conçue de préférence sous la forme d'une bosse, que la butée (16) est réglable en direction longitudinale du boîtier (11) et liée à celui-ci et que la butée (16) est formée sur un tiroir (31) comprenant un évidement (32) sous la forme d'un trou oblong et qui, moyennant des cames de crantage (33, 34) prévus sur le boîtier (11) et s'engrenant dans l'évidement (32), se laisse positionner à diverses distances de l'ouverture de distribution (15).

2. Dispositif (1) de distribution de rouleaux de ouate (10) ou de pièces similaires en barre, logés dans un boîtier cube (11), le boîtier (11) comportant sur un des côtés étroits (43) une ouverture de distribution (15) disposée parallèlement aux rouleaux de ouate enfermés (10), et le fond (12) du boîtier (11) portant les rouleaux de ouate (10) étant réglable en hauteur dans le boîtier (11), ceci contre la force d'un ou de plusieurs ressorts de rappel (17, 17') et dont un côté étroit passe partiellement ou entièrement par l'ouverture de distribution (15), et également selon la revendication 1,
caractérisé en ce que
pour le réglage en hauteur du fond (12), il est prévu en tant qu'élément de réglage le couvercle (14) du boîtier (11), qui agit sur celui-ci soit directement soit via des éléments intermédiaires (26), ou qui est prévu sur celui-ci.

3. Dispositif d'après les revendications 1 ou 2,
caractérisé en ce
qu'une extrémité du fond (12) est articulée sur le boîtier (11) ou menée réglable en hauteur dans le boîtier (11).

4. Dispositif d'après une ou plusieurs des revendications précédentes,
caractérisé en ce que
le fond (12) est conçu sous la forme d'une pièce angulaire (21) limitant la chambre de réception (13) du boîtier (11) et que son bras (22) opposé à l'ouverture de distribution (15) est articulé au moyen d'un boulon d'articulation (19) à l'endroit du bord supérieur du boîtier (11).

5. Dispositif d'après la revendication 2,
caractérisé en ce que,
en tant qu'élément de réglage, il est prévu un couvercle (14) posé sur le boîtier (11) qui est articulé avec le fond (12) sur le boîtier (11) et qui, moyennant une saillie (26) ou un organe semblable, collabore en tant qu'élément intermédiaire avec le fond (12).

6. Dispositif d'après la revendication 5,
caractérisé en ce que
l'élément de réglage est formé par une poignée (28) sortant du boîtier (11) et prévue sur le fond (12), de préférence à l'endroit du boulon d'articulation (19) qui le porte.

7. Dispositif d'après une ou plusieurs des revendications 1 à 6,
caractérisé en ce que,
sur l'extrémité sortant de l'ouverture de distribution (15), il est formé sur le fond (12) une cuvette réceptrice (25) pour un rouleau de ouate (10'), cette cuvette étant disposée devant le boîtier (11).

8. Dispositif d'après une ou plusieurs des revendications 1 à 6,
caractérisé en ce que
le boîtier (11) est muni d'une cuvette réceptrice solidaire (34) disposée devant l'ouverture de distribution (15).

9. Dispositif d'après une ou plusieurs des revendications 1 à 8,
caractérisé en ce que
devant l'ouverture de distribution (15) du boîtier (11), le fond (12) est muni d'une déformation de préférence convexe (24) saillant dans la chambre de réception (13).

10. Dispositif d'après la revendication 9,
caractérisé en ce que
la déformation (24) prévue sur le fond (12) est disposée à une distance de l'ouverture de distribution (15) qui correspond à peu près au diamètre des rouleaux de ouate (10) contenus dans le boîtier (11).

11. Dispositif d'après une ou plusieurs des revendications 1 à 10,
caractérisé en ce que
sur une ou sur les deux faces intérieures des deux parois latérales (41, 42) du boîtier (11), il est prévu une ou plusieurs saillies (20) sous la forme de coins, qui s'étendent de préférence sur la plage de réglage du fond (12).

12. Dispositif d'après la revendication 1,
caractérisé en ce que
la butée (16) est munie d'un évidement (36) recevant la cuvette collectrice (25) prévue sur le fond (12).

13. Dispositif d'après la revendication 1,
caractérisé en ce que
la paroi du boîtier (11) orientée vers la butée (16), collaborant avec le fond (12) et limitant l'ouverture de distribution (15), est conçue sous la forme d'une surface oblique (27) inclinée vers l'extérieur.

14. Dispositif d'après une des revendications 1, 12 ou 13,
caractérisé en ce que
sur sa face éloignée du boîtier (11), il est prévu sur la butée (16) une cuvette collectrice (36).

15. Dispositif d'après une ou plusieurs des revendications 1 à 14,
caractérisé en ce que
dans le cas d'un fond (12) conçu sous la forme d'une pièce angulaire (21), les ressorts de rappel (17) agissant sur le fond (12) du boîtier (11) sont montés entre son bras articulé (22) et le boîtier (11).

16. Dispositif d'après une ou plusieurs des revendications 1 à 15,
caractérisé en ce que
le ressort de rappel (17') agissant sur le fond (12) est conçu sous la forme d'un ressort à lame coulé sur celui-ci.
